# EUROPEAN PATENT APPLICATION

(11) **EP 4 261 840 A2**
(43) Date of publication of application: **18.10.2023**
(21) Application number: 23167387.2
(22) Date of filing: 11.04.2023
(51) Int. Cl.: G16H 30/40, G16H 30/20, H04L 9/40, H04W 12/02

(54) **REAL TIME CLOUD IMAGE PROCESSING FOR ENDOSCOPY ANALYSIS**

(30) Priority: 11.04.2022 US 202263329638 P
(71) Applicant: Odin Vision, London W1W7TS (GB)
(72) Inventor: MOUNTNEY, Peter, London (GB); TOTH, Daniel, London (GB); RIVIERE, Vincent, London (GB); HEBBAR, Sanjith, London (GB)
(74) Representative: Gillott-Jones, Nathan Philip

(57) **Abstract**

A method for image analysis includes receiving compressed, encrypted image data from an imaging session at a healthcare location, unencrypting the image data, and identifying a characteristic of interest in the compressed image data. The method further includes annotating the compressed image data reflecting the identified characteristic of interest to generate computer annotations and streaming the computer annotations to the healthcare location as the imaging session is in progress.

## Description

### CROSS-REFERENCE TO RELATED APPLICATIONS

The present application claims the benefit of priority to U.S. Provisional Patent Application No. 63/329,638, filed April 11, 2022, entitled "REAL TIME CLOUD IMAGE PROCESSING FOR ENDOSCOPY ANALYSIS."

### TECHNICAL FIELD

The present application relates to cloud based systems for real time analysis of medical images.

### BACKGROUND

Analysis of medical images may utilize expensive and specialized computing equipment (e.g., specialized computer servers), which may be impracticable to maintain at a healthcare location. Real-time medical image processing (e.g., computer annotation of images as an imaging procedure is ongoing) is often very helpful to providers. Such real-time image processing is often underutilized or impossible due to expensive equipment and lag when using, for example, remote computing resources to conduct image processing.

### SUMMARY

An example method disclosed herein includes receiving compressed, encrypted image data from an imaging session at a healthcare location, unencrypting the image data, and identifying a characteristic of interest in the image data. The method further includes annotating the compressed image data reflecting the identified characteristic of interest to generate computer annotations and streaming the computer annotations to the healthcare location as the imaging session is in progress.

An example system disclosed herein includes local computing resources configured to compress image data received from an imaging device where the image data is collected during an imaging procedure. The system further includes an image processing service hosted in a cloud computing environment configured to receive compressed image data from the local computing resources, analyze the compressed images to provide computer annotations to the compressed images, and transmit the computer annotations to the local computing resources for display as the imaging procedure is ongoing.

An example method disclosed herein includes detecting a beginning of an imaging procedure at a healthcare location and analyzing, at a cloud location, an image stream produced during the imaging procedure. The method further includes incrementing a procedure count associated with the healthcare location.

Additional embodiments and features are set forth in part in the description that follows, and will become apparent to those skilled in the art upon examination of the specification and may be learned by the practice of the disclosed subject matter. A further understanding of the nature and advantages of the present disclosure may be realized by reference to the remaining portions of the specification and the drawings, which form a part of this disclosure. One of skill in the art will understand that each of the various aspects and features of the disclosure may advantageously be used separately in some instances, or in combination with other aspects and features of the disclosure in other instances.

### BRIEF DESCRIPTION OF THE DRAWINGS

The description will be more fully understood with reference to the following figures in which components are not drawn to scale, which are presented as various examples of the present disclosure and should not be construed as a complete recitation of the scope of the disclosure, characterized in that:
FIG. 1 illustrates an example environment utilizing real time cloud based image analysis in accordance with various embodiments of the disclosure.
FIG. 2 illustrates a schematic diagram of local compute resources in communication with a computing environment to access image processing in accordance with various embodiments of the disclosure.
FIG. 3 illustrates a schematic diagram of an image processing service in accordance with various embodiments of the disclosure.
FIG. 4 is a schematic diagram of a computing system which may be used to implement various embodiments in the examples described herein.
FIG. 5 is a flow diagram of an example method for utilizing an image processing service in accordance with various embodiments of the disclosure.
FIG. 6 is a flow diagram of an example method of performing image processing by an image processing service in accordance with various embodiments of the disclosure.
FIG. 7 is a flow diagram of an example method of counting procedures originating from a healthcare facility utilizing an image processing service in accordance with various embodiments of the disclosure.

### DETAILED DESCRIPTION

The system described herein generally provides real time image processing for medical images using a cloud based computing system. Real time image processing is helpful for medical providers, as it can provide additional information to the provider while the patient is still being imaged. For example, in various imaging modalities, a provider interprets images as the images are collected to make decisions relating to disease detection, diagnosis, therapy, and/or navigation. Accordingly, real time image processing and analysis may assist physicians in making such decisions. For example, it may be easier to obtain additional images of areas of interest identified by the real time image processing. Such real time processing uses large amounts of computing resources. Accordingly, using local computing resources to process medical images can be cost prohibitive and impractical. Cloud based image analysis may provide flexible access to the computing resources used to process such images. However, images streams are generally sent over a network to cloud based image processing systems. To ensure real time (or near real time) image processing (e.g., processing with a delay of, for example, less than 500 ms), image streams are generally compressed for transmission to cloud based image processing systems. Real time, or near real time, image processing may be image processing completed without a perceptive delay by a user.

The real time, cloud based image processing system disclosed herein provides computing power through taking advantage of remote compute resources, while still enabling real time image analysis. Real time image analysis (or near real time image analysis) may be provided by compression of images and, in some examples, analysis of the compressed images at a cloud based image processing service. Further, in some examples, the cloud based image processing service may be structured using a microservice architecture, allowing for scalability (e.g., access to more compute resources when needed), sharing of the image processing service by multiple healthcare sites, and more efficient use of compute resources. For example, the cloud based image processing service described herein may work in cooperation with local compute systems. The local compute system may compress and encrypt image data, ultimately streaming the image data to the cloud based image processing service. When the cloud based imaging service receives the image stream, the service may analyze the compressed image and return the result (e.g., computer annotations) to the local compute system, where the analysis is displayed for an end user.

Further, hosting the image processing service at a cloud computing environment may simplify the process of medical device regulation. For example, when software is regulated as a medical device, each installation of the software may be associated with a unique identifier number (e.g., fingerprint or other indicator). Software is tracked by the unique identifier such that defective software is tracked. In some instances, software may be hosted in a cloud environment, but with a different installation for each customer or user. The image processing service described herein may be scalable to serve multiple customers. Accordingly, the software making up the image processing service may have one identifier for purposes of tracking the software as a medical device. Using one identifier simplifies updates, as an update to the image processing service only has to be made at one location. Further, use of one identifier means that multiple installations do not need to be managed or tracked, ultimately saving time and resources. Further, by doing analysis directly on lossy data, the analysis can be completed faster, such that the analysis can be completed in the cloud while still being viewable during the imaging procedure. The unique identifiers may be applied before or after transmission to the cloud (e.g., at a healthcare location).

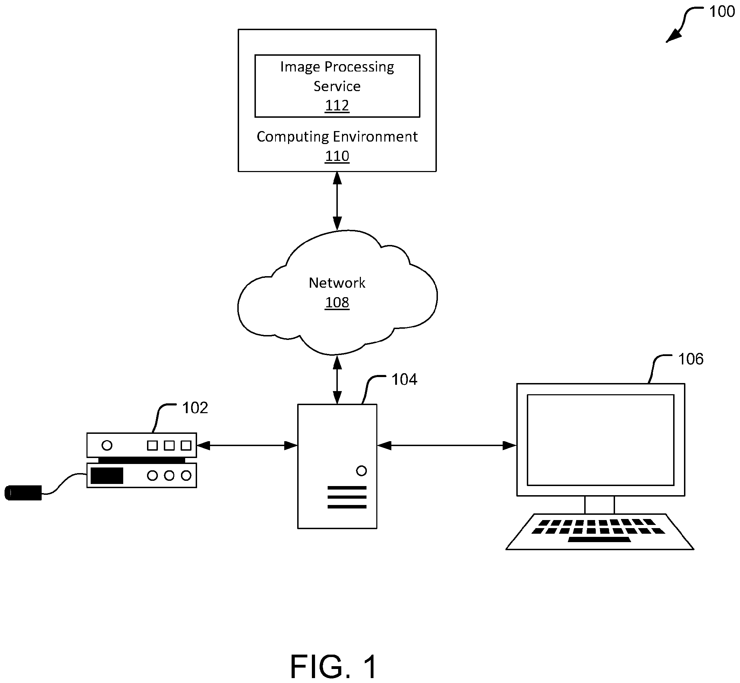

Various embodiments of the present disclosure will be explained below in detail with reference to the accompanying drawings. Other embodiments may be utilized, and structural, logical and electrical changes may be made without departing from the scope of the present disclosure. FIG. 1 illustrates an example environment utilizing real time cloud based image analysis in accordance with various embodiments of the disclosure. An imaging device 102 and a display 106 may communicate with a local computer 104 to access a computing environment 110 including image processing service 112 via a network 108. In various examples, the imaging device 102, local computer 104, and display 106 may be located at a healthcare location (e.g., a hospital, physician's office, or the like), while the computing environment 110 may be a cloud computing environment remote from the healthcare location.

The imaging device 102 may be a medical imaging device configured to collect images of patients (either interior or exterior images), which may be used for diagnostic purposes. The imaging device 102 may, in some examples, be a device for collecting images which may be streamed to the image processing service 112, ultrasound images, endoscopy images, laparoscopic images, fluoroscopy images, and the like. In various examples, the imaging device 102 may be a device for collecting images which may be stored and/or recorded before being sent to the image processing service 112. For example, the imaging device 102 may be an magnetic resonance imaging (MRI) machine, computed tomography (CT) machine, X-Ray machine, and the like. In some examples, the imaging device 102 may include, or be in communication with, a trigger device. For example, a footswitch may be connected to the imaging device 102 and, when a user provides input through the footswitch, an imaging session is initiated at the imaging device 102 and the image processing service 112.

The local computer 104 may be a desktop computer, laptop computer, local server, or the like. Generally, the local computer 104 is connected to the network 108, the imaging device 102, and the display 106. For example, the local computer 104 may receive image data (e.g., image streams) from the imaging device 102, anonymize, compress, and/or encrypt the image data, send the image data to the image processing service 112 over the network 108, receive processed image data from the image processing service 112 over the network 108, and present the processed image data to a user (e.g., a physician) via the display 106. The display 106 may, in some examples, be a part of the local computer 104. In some examples, the display 106 may be part of, or connected to, the imaging device 102. In various examples, the display 106 may be a liquid crystal display (LCD), light emitting diode (LED) display, touchscreen, and the like.

The network 108 may be implemented using one or more of various systems and protocols for communications between computing devices. In various embodiments, the network 108 or various portions of the network 108 may be implemented using the Internet, a local area network (LAN), a wide area network (WAN), and/or other networks. In addition to traditional data networking protocols, in some embodiments, data may be communicated according to protocols and/or standards including near field communication (NFC), Bluetooth, cellular connections, and the like. Various components of the system 100 may communicate using different network protocols or communications protocols based on location. For example, components of the image processing service 112 may be hosted within a cloud computing environment and may communicate with each other using communication and/or network protocols used by the cloud computing environment.

The computing environment 110 may be a cloud computing environment, including computing resources in several physical locations. Accordingly, the image processing service 112 may, in various examples, be composed of various microservices distributed across various compute resources. In some examples, the computing environment 110 may include computing resources in multiple cloud environments, such as proprietary cloud environments (e.g., dedicated servers), shared or public cloud environments, etc.

In various implementations, the computing environment 110 may include or utilize one or more hosts or combinations of compute resources, which may be located, for example, at one or more servers, cloud computing platforms, computing clusters, and the like. Generally, the image processing service 112 is implemented by the computing environment 110, which includes compute resources including hardware for memory and one or more processors. For example, the computing environment 110 may utilize or include one or more processors, such as a CPU, GPU, and/or programmable or configurable logic.

In some embodiments, various components of the computing environment 110 may be distributed across various computing resources, such that the components of the computing environment 110 communicate with one another through the network 108 or using other communications protocols. For example, in some embodiments, the computing environment 110 may be implemented as a serverless service, where computing resources for various components of the computing environment 110 may be located across various computing environments (e.g., cloud platforms) and may be reallocated dynamically and automatically according to resource usage of the image processing service 112. In various implementations, the image processing service 112 may be implemented using organizational processing constructs such as functions implemented by worker elements allocated with compute resources, containers, virtual machines, and the like.

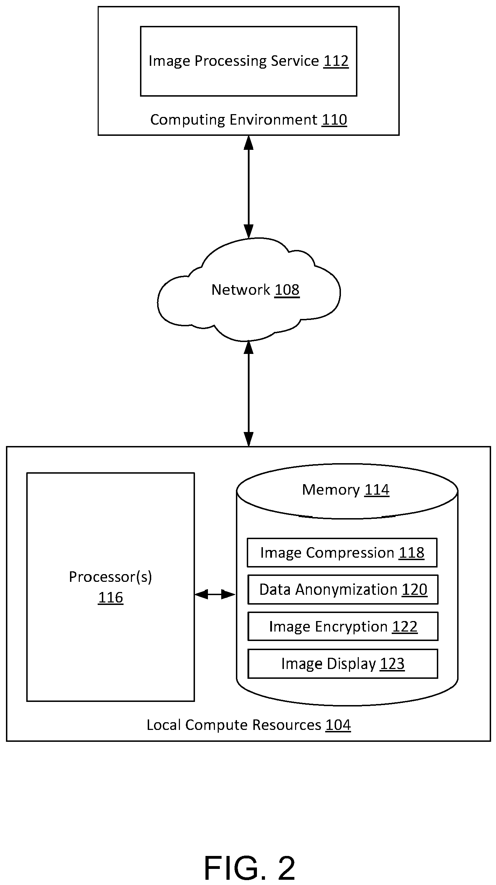

FIG. 2 illustrates a schematic diagram of local compute resources 104 in communication with a computing environment 110 to access the image processing service 112 in accordance with various embodiments of the disclosure. To utilize the image processing service 112, the local compute resources 104 may do some formatting and/or processing image stream data before streaming image data to the image processing service 112. For example,

Generally, local compute resources 104 may include a desktop or laptop computer, mobile device, local server, or other local combinations of processing and memory resources. In various implementations, the compute resources 104 and/or additional compute resources may be implemented using any number of computing devices included, but not limited to, a desktop computer, a laptop, tablet, mobile phone, smart phone, wearable device (e.g., AR/VR headset, smart watch, smart glasses, or the like), smart speaker, vehicle (e.g., automobile), or appliance. Generally, the local compute resources 104 may include one or more processors, such as a central processing unit (CPU) and/or graphics processing unit (GPU). The compute resources 104 may generally perform operations by executing executable instructions (e.g., software) using the processors. In various embodiments, multiple local compute resources 104 may be provided with access to the image processing service 112, where local compute resources 104 may be located at one or several locations. For example, the image processing service 112 may be accessible by local compute resources 104 at several different hospitals and/or physician's offices. Where multiple local compute resources 104 access the image processing service 112, different local compute resources 104 may be provided with varying permission, settings, and the like, and may be authenticated by an authentication service prior to accessing the image processing service 112.

Generally, the local compute resources 104 include compute resources including hardware for memory 114 and one or more processors 116. For example, the local compute resources 104 may utilize or include one or more processors, such as a CPU, GPU, and/or programmable or configurable logic. In some embodiments, various components of the local compute resources 104 may be distributed across various computing resources, such that the components of the local computing environment 104 communicate with one another through wired connections, the network 108, or using other communications protocols.

The memory 114 may include instructions, which, when executed by processors 116, perform various functions to stream image data from the local computing system 104 to an image processing service 112. For example, the memory 114 may include instructions for image compression 118, data anonymization 120, image encryption 122, and image display 123. Similar to the processor 116, memory resources 114 included in the local computing system 104 may be distributed across various physical computing devices.

When executed by the processors 116, the instructions for image compression 118 may compress image data (e.g., images or image streams) before sending the image data to the image processing service 112, enabling the image processing service 112 to handle images of various resolution (e.g., standard definition, high definition, 4k, and/or 8k). Higher resolution images may contain too much information to stream the images to the image processing service 112 via the network 108. In some examples, instructions for image compression 118 may crop an input image to a region that contains only useful information (e.g., only a region including an organ or structure of interest). The cropped portion of the image may then be compressed using lossy or lossless compression techniques. When compressed using lossy compression techniques, enough data may be captured to allow for analysis of the lossy data at the imaging processing service 112. In some examples, compression may be performed using neural networks or standard compression techniques, such as deflate, discrete cosine transform, or wavelet transform.

In some examples, compression may be performed using an autoencoder network. For example, an autoencoder may utilize an encoder and decoder, where the encoder compresses input data to reduce its dimensionality and the decoder attempts to reconstruct the original data from the compressed data. The autoencoder may use residual blocks, where each of the residual blocks includes a convolutional layer, a batch normalization layer, and an activation layer followed by another convolutional layer and batch normalization layer. For each residual block, the output of the block is concatenated with the input and passed onto the next layer. In some examples, the autoencoder network includes an encoder with three downscaling units, where each downscaling unit is followed by three residual blocks and a decoder with three upscaling units, where each upscaling unit is followed by three residual blocks. A downscaling unit generally includes a convolutional layer, a batch normalization layer, and a parametric rectified linear unit (ReLU) activation unit. An upscaling unit generally includes a deconvolutional layer, a batch normalization layer, and a parametric ReLU activation unit.

In some examples, when an autoencoder network is used to perform the compression, an input video stream (e.g., including image data) is broken down into image frames and passed through the encoder sub-network of the autoencoder network, which may be located at the local compute resources 104. The encoder sub-network outputs a compressed latent object, which is transmitted to the cloud server (e.g., the image processing service 112). The decoder sub-network may be present at the image processing service 112 and may construct the original input image from the compressed latent object. The reconstructed image may then be passed to other components of the image processing service 112 for analysis, and the results of such analysis (e.g., computer annotation of the image) may be streamed back to the local compute resources 104.

In other examples, an autoencoder using encoding layers may perform image compression, and the image processing service 112 may analyze the compressed data directly. For example, compression may be performed by an encoder network at local compute resources 104, and the resulting compressed latent object may be transmitted to the image processing service 112. The image processing service 112 may include components (e.g., detectors) trained to perform analysis directly on the compressed data (e.g., without decompression by a decoder sub-network). The results of such analysis (e.g., computer annotation of the image) may be streamed back to the local compute resources 104.

When executed by the processors 116, the instructions for data anonymization 120 may remove identifying information from image data before transmitting image data to the image processing service 112. For example, patient identifiable information or other confidential information may be in image data or metadata captured along with the image. Data anonymization 120 may review image data and delete metadata before transmission. Data anonymization 120 may further locate and remove other types of confidential information. For example, confidential information can include text written into an image (e.g., patient name, identifier, or other information) or faces or other identifiable features of patients (e.g., before a scope is inside of the body). To remove this type of confidential information, data anonymization 120 may take several approaches. Instructions for data anonymization 120 may include optical character recognition to identify the location of any text in the image. Such text may then be removed before transmission. The instructions may further include a facial recognition algorithm which may be run on the image data to identify faces. In some examples, where a face is included in the image, the image is not transmitted to the image processing service 112 for further processing. The instructions may further include a neural network trained to classify an image as either inside of the body or outside of the body. In some examples, where an image is classified as being outside of the body, the image is not transmitted to the image processing service 112 for further processing.

When executed by the processors 116, the instructions for image encryption 122 may encrypt the image data before the image data is streamed (e.g., transmitted) to the image processing service 112. Image encryption 122 may allow images to be securely transmitted from local compute resources 104 via the network 108 to the image processing service 112. For example, image encryption 122 may encrypt data based on the TLS specification to transfer data based on various protocols, such as HTTPS or WSS protocols. In some examples, the instructions for image encryption 122 may further unencrypt results received at the local compute resources 104 from the image processing service 112. In various examples, unecrypting results may include checking the ID and frame information for integrity and storing such information.

When executed by the processors 116, the instructions for image display 123 may receive the results from the image processing service 112 and visualize the results by overlaying results on a separate layer or canvas from the original image stream. In some examples, image display 123 may overlay computer annotation over the image stream using a coordinate system shared between the computer annotations and the image stream. Image display 123 may further stream raw image data from the imaging device 102 to the display 106. For example, image display 123 may split an incoming stream from the imaging device 102 by reading the stream into a video capture card, and then splitting the stream into two streams. The first stream may be displayed directly onto the display 106. The second stream may be sent to the image processing service 112.

When results are received from the image processing service 112, the results may be displayed with the first, original image stream using alpha blending. In some examples, the second layer may have an alpha channel, such that the second layer is transparent when no additional information is present in the second layer. As the visualization layers may be independent, information may be displayed in an asynchronous manner, performance of the original image stream may be unaffected by the visualization of the additional information in the second layer. In some examples, when results (e.g., computer annotations) are received from the image processing service 112, image display 123 may determine whether the results were received within a threshold period of time. If not, the image display 123 may determine that the results are too old to be useful (e.g., there would be a perceptible delay to the user) and may not display the results overlaid on the first stream. The image display 123 may then display the next results received from the image processing service 112.

In some examples, the local computing resources and/or the image processing service 112 may further include internet and/or IT network performance monitoring. Such monitoring may be useful as slow or decreased network performance may delay image analysis. For example, a network health monitor may send regular data packets from the client to a server (e.g., the computing environment 110) and measures if and when a response to the data packets is received. Such response time may be used to approximate a response time for receiving output from the image processing service 112. A measure of connection speed may be displayed at the display 106. For example, the response time may be displayed to the end user by colored bars, where the higher the response time, the fewer bars are displayed. For example, a higher number of bars may indicate that the client has a relatively fast connection to the image processing service 112 (e.g., with a response time less than 33 ms). In some examples, network performance monitoring may further monitor response times for connections to individual modules and/or microservices of the image processing service 112.

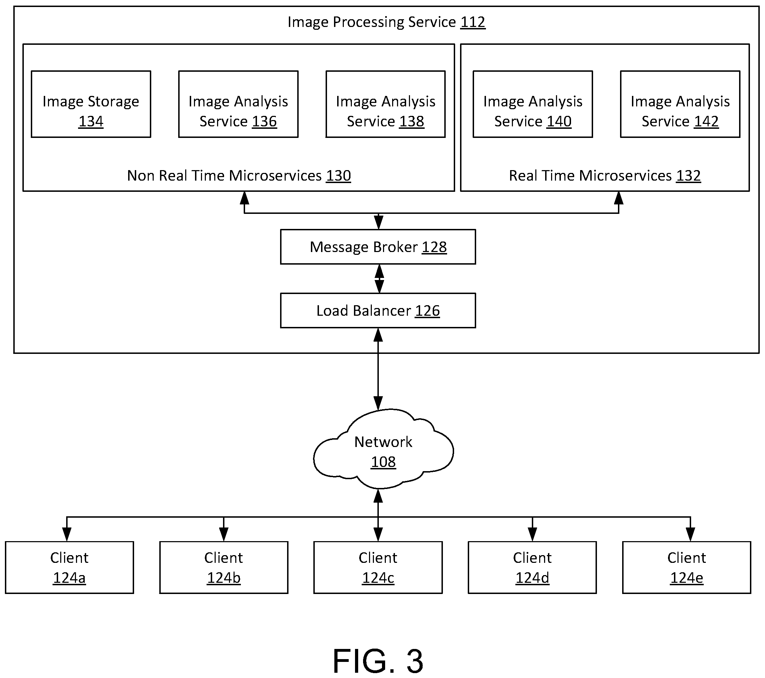

FIG. 3 illustrates a schematic diagram of an image processing service 112 in accordance with various embodiments of the disclosure. As shown in FIG. 3, the image processing service 112 may serve multiple clients 124a-e. Clients 124a-e may be located at different healthcare locations (e.g., hospitals, physician's offices, etc.) and/or may be affiliated with different providers, healthcare organizations, hospital systems, and the like. To access the image processing service 112, a client (e.g., client 124a) may connect to a load balancer 126 of the image processing service 112 via the network 108. The load balancer 126 may determine, in some examples, how to direct the request, whether to scale the image processing service 112 (e.g., whether to add new instances of a microservice to accommodate client requests), and the like. In some examples, the client 124a may go through an authentication service and/or other type of login before being directed to the load balancer 126. The image stream and/or the client 124a may then be directed to a message broker 128 to ultimately connect to the correct image processing service based on the request type, image type, or the like.

In various examples, the message broker 128 and/or other components of the image processing service 112 may generate a unique fingerprint for each frame or individual image of the image stream received from the user device 102. For example, the message broker 128 may generate a unique identifier for each frame of an image stream. The unique identifier may identify the user device 102, the order of the frame within the image stream, and the like. In some examples, the unique identifier may generated at least in part based on a timestamp (e.g., a timestamp reflecting when the frame was generated), a clock, a counter, or other hardware components or metadata associated with the frame. Such unique identifiers may be used to track the frames of the stream during analysis to keep the frames in an order reflecting the order of generation. The unique identifiers may further be used, in various examples, to track usage of the image processing service 112 by individuals or organizations, to pair annotations with frames of an image stream, and the like.

In some examples, a unique identifier and/or fingerprint may be generated at the healthcare location and may be transmitted with frames of the stream to the image processing service 112. In such examples, the unique identifiers may be utilized by the image processing service 112 to maintain an order of the frames (e.g., an order that the frames were received at the image processing service 112). Such unique identifiers generated at the healthcare location may further be utilized by the image processing service 112 to generate unique identifiers for annotations to the imaging frames.

In various examples, the image processing service 112 (e.g., the load balancer 126) may track access to the image processing service 112 by a specific client, a group of clients (e.g., clients associated with a physician, healthcare system, or the like), or other grouping. Such tracking may allow the system to keep track of how many imaging procedures are performed using the image processing service 112, which may be useful to bill users for use of the image processing service 112. For example, the image processing service 112 may utilize a counter, which tracks number of procedures based on a user ID, which may be provided by a user, for example, at login to the image processing service 112. When a new procedure is started with the user ID, a counter associated with the user ID may be incremented.

In various examples, tracking may also be utilized by the load balancer 126 to determine if and how to scale the resources of the image processing service 112. For example, the load balancer 126 may add a new instance of a microservice to the image processing service 112 when the number of requests exceeds the capacity of the image processing service 112. In some examples, the load balancer 126 may track usage of the image processing service 112 to identify patterns, and may scale the resources of the image processing service 112 based on the patterns. For example, the load balancer 126 may identify a lower pattern of use at nighttime, and may scale in or scale down the image processing service 112 (e.g., by terminating instances of the microservices of the image processing service 112) during such times of reduced demand.

As shown in FIG. 3, in various examples, the image processing service 112 may be composed of other services (e.g., microservices) performing some function for the image processing service 112. The microservices may include real time microservices 132 (e.g., services with less than 2 ms of latency) and non real time microservices 130 (e.g., services with less than a second of latency). Some examples of microservices utilized by the image processing service 112 may include, for example, image storage, detection of specific elements in images (e.g., cecum detection, polyp detection), estimation of specific elements in images (e.g., visible mucosa estimation), and/or diagnosis of specific elements in images (e.g., polyp diagnosis). In some examples, additional microservices may be started or stopped based on the types of requests being made by clients 124a-e to the image processing service 112.

The image processing service 112 may include microservices that analyze compressed image data. Such microservices may include, in various examples, machine learning models trained to detect specific structures, characteristics of images, or the like. In some examples, such models may be trained using lossy data, such that the models can ultimately analyze compressed images. Such lossy compression may retain enough information within images to perform meaningful analysis of the images. Conventionally, image analysis is done on uncompressed images, as image analysis uses as much information as possibly for accuracy. Where the image processing service 112 analyzes compressed image data, the images are compressed and the models are trained such that the compressed images contain enough data for accurate analysis by the image processing service 112.For example, the models may be trained using compressed image data so that the model may be used to analyze compressed image data. Compressed images may have less visual information, such that it may be harder to analyze compressed images. Where a model is trained on uncompressed images and analyze compressed images, performance may be worse (e.g., fewer features may be correctly identified) than a model trained on compressed images to analyze compressed images.

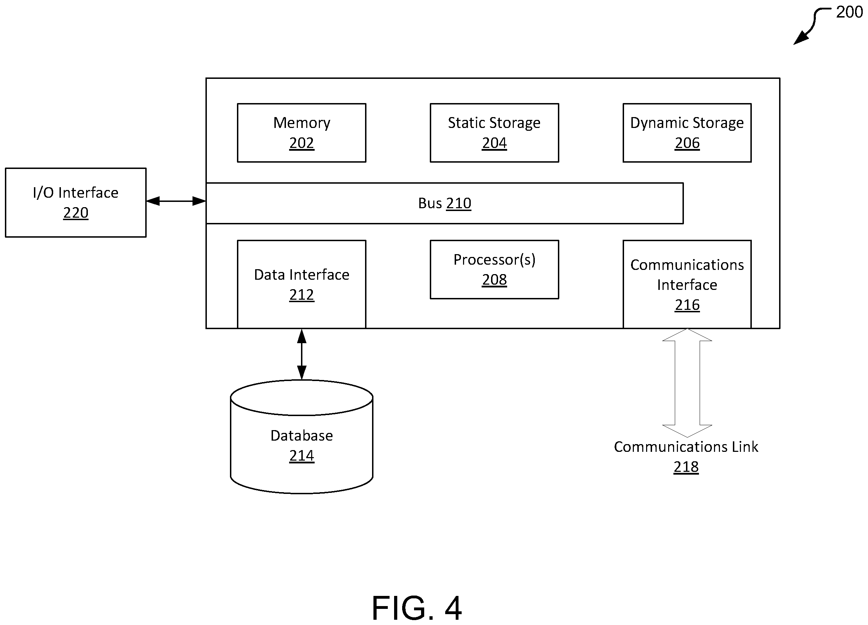

FIG. 4 is a schematic diagram of a computing system 200 which may be used to implement various embodiments in the examples described herein. For example, local compute resources 104 may be located at one or several computing systems 200. In various embodiments, computing environment 110 is also implemented by a computing system 200. This disclosure contemplates any suitable number of computing systems 200. For example, a computing system 200 may be a server, a desktop computing system, a mainframe, a mesh of computing systems, a laptop or notebook computing system, a tablet computing system, or a combination of two or more of these. Where appropriate, the computing system 200 may include one or more computing systems; be unitary or distributed; span multiple locations; span multiple machines; span multiple data centers; or reside in a cloud, which may include one or more cloud components in one or more networks.

Computing system 200 includes a bus 210 (e.g., an address bus and a data bus) or other communication mechanism for communicating information, which interconnects subsystems and devices, such as processor 208, memory 202 (e.g., RAM), static storage 204 (e.g., ROM), dynamic storage 206 (e.g., magnetic or optical), communications interface 216 (e.g., modem, Ethernet card, a network interface controller (NIC) or network adapter for communicating with an Ethernet or other wire-based network, a wireless NIC (WNIC) or wireless adapter for communicating with a wireless network, such as a WI-FI network), input/output (I/O) interface 220 (e.g., a keyboard, keypad, mouse, microphone). In particular embodiments, the computing system 200 may include one or more of any such components.

In particular embodiments, processor 208 includes hardware for executing instructions, such as those making up a computer program. The processor 208 circuitry includes circuitry for performing various processing functions, such as executing specific software for performing specific calculations or tasks. In particular embodiments, I/O interface 220 includes hardware, software, or both, providing one or more interfaces for communication between computing system 200 and one or more I/O devices. Computing system 200 may include one or more of these I/O devices, where appropriate. One or more of these I/O devices may enable communication between a person and computing system 200.

In particular embodiments, communications interface 216 includes hardware, software, or both providing one or more interfaces for communication (such as, for example, packet-based communication) between computing system 200 and one or more other computer systems or one or more networks. One or more memory buses (which may each include an address bus and a data bus) may couple processor 208 to memory 202. Bus 210 may include one or more memory buses, as described below. In particular embodiments, one or more memory management units (MMUs) reside between processor 208 and memory 202 and facilitate accesses to memory 202 requested by processor 208. In particular embodiments, bus 210 includes hardware, software, or both coupling components of computing system 200 to each other.

According to particular embodiments, computing system 200 performs specific operations by processor 208 executing one or more sequences of one or more instructions contained in memory 202. For example, instructions for image compression 118, data anonymization 120, image encryption 122, and image display 123 may be contained in memory 202 and may be executed by the processor 208. Such instructions may be read into memory 202 from another computer readable/usable medium, such as static storage 204 or dynamic storage 206. In alternative embodiments, hard-wired circuitry may be used in place of or in combination with software instructions. Thus, particular embodiments are not limited to any specific combination of hardware circuitry and/or software. In one embodiment, the term "logic" shall mean any combination of software or hardware that is used to implement all or part of particular embodiments disclosed herein.

The term "computer readable medium" or "computer usable medium" as used herein refers to any medium that participates in providing instructions to processor 208 for execution. Such a medium may take many forms, including but not limited to, nonvolatile media and volatile media. Non-volatile media includes, for example, optical or magnetic disks, such as static storage 204 or dynamic storage 206. Volatile media includes dynamic memory, such as memory 202.

Computing system 200 may transmit and receive messages, data, and instructions, including program, e.g., application code, through communications link 218 and communications interface 216. Received program code may be executed by processor 208 as it is received, and/or stored in static storage 204 or dynamic storage 206, or other storage for later execution. A database 214 may be used to store data accessible by the computing system 200 by way of data interface 212.

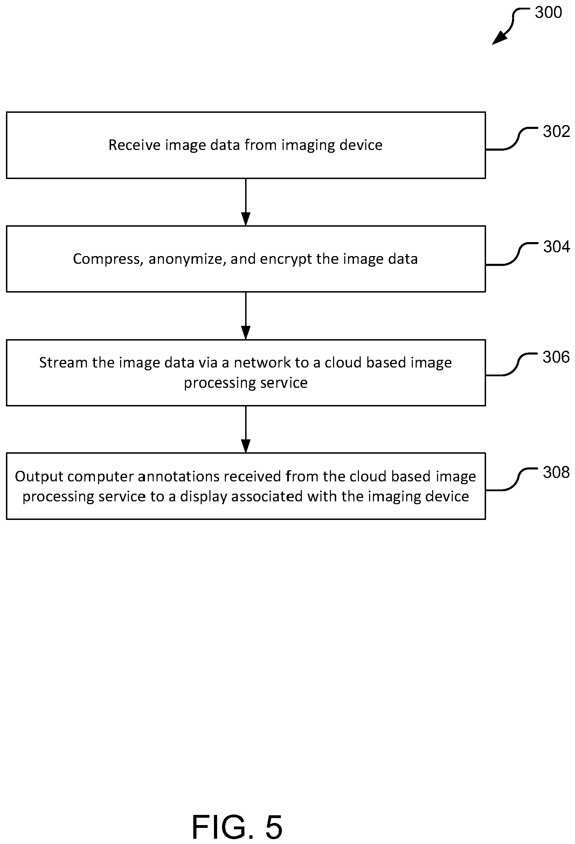

FIG. 5 is a flow diagram of an example method 300 for utilizing an image processing service. Local compute resources 104 receive imaging data from an imaging device 102 at block 302. In some examples, the imaging data may be received through a wired or wireless connection between the imaging device 102 and the local compute resources 104. In various examples, the process of transmitting image data from the imaging device 102 to the local compute resources 104 may be initiated by a user (e.g., a physician or other provider) using a button, switch, or other method at the imaging device 102. In some examples, image display 123, or other components of the local compute resources 104 may receive a stream from the imaging device 102 and may split the stream, displaying a first portion of the stream in real-time and process a second portion of the stream before ultimately providing the stream to the image processing service 112.

At block 304, the local compute resources 104 compress, anonymize, and encrypt the image data. For example, instructions for data anonymization 120 may remove identifying information (e.g., patient name or other identifying text) from the image data using optical character recognition or other methods of detecting text. Image compression 118 may then encrypt the anonymized image data using lossy or non-lossy image compression techniques. In various examples, image compression 118 may utilize an autoencoder for compression. Such an autoencoder may be trained using backpropagation to provide output as similar to the input as possible. Image encryption 122 may then encrypt the anonymized, compressed image data before streaming the image data to the image processing service 112. The local compute resources 304 stream the image data via a network 108 to a cloud based image processing service 112 at block 306.

At block 308, the local compute resources 104 output computer annotations received from the cloud based image processing service to a display associated with the imaging device 102. After transmitting the image data to the cloud based image processing service 112, the local compute resources 104 may receive results from the cloud based image processing service 112. In various examples, when results are received from the image processing service 112, image display 123 of the local compute resources may display results with the first, original image stream using alpha blending. In some examples, the second layer may have an alpha channel, such that the second layer is transparent when no additional information is present in the second layer. As the visualization layers may be independent, information may be displayed in an asynchronous manner, performance of the original image stream may be unaffected by the visualization of the additional information in the second layer.

Results may include, for example, computer annotations for the image data originally sent to the cloud based image processing service 112. Computer annotations may identify structures in the image, show information about structures in or characteristics of the image such as, for example, measurements of structures in the image. In some examples, annotations may include flags noting areas of interest in the image, bounding boxes enclosing regions of interest, risk results, indications that the provider has reached a certain part of the anatomy, and the like. Such computer annotations may be associated with a coordinate system of the image data such that the computer annotations, when viewed in conjunction with the image stream, appear in the correct location.

In various examples, the computer annotations may be displayed at block 308 in near real-time (e.g., slightly delayed) with respect to the image stream. For example, computer annotations may be displayed overlaid on a later image frame than the image data used to generate the annotations. The later image frame may be collected close enough in time to the image data used to generate the annotations, such that the delay is largely imperceptible to the operator.

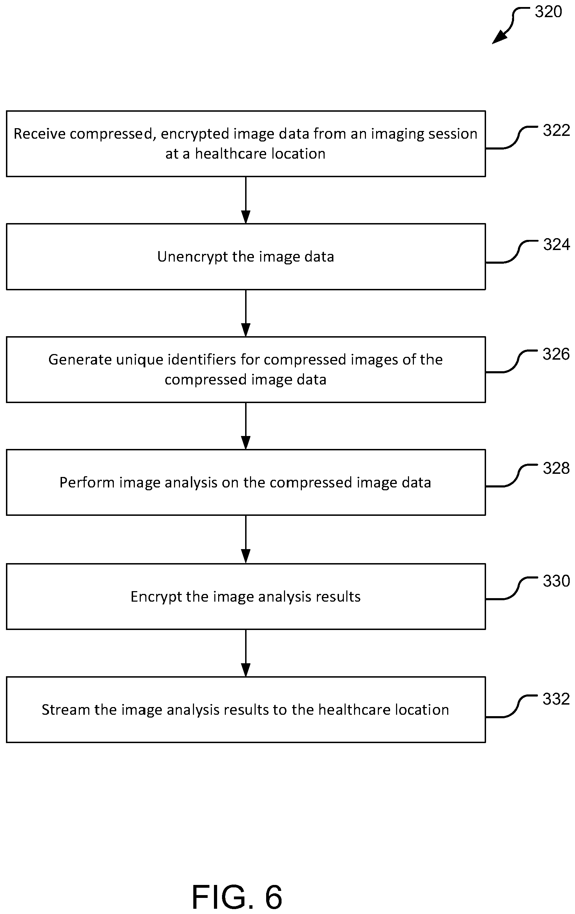

FIG. 6 is a flow diagram of an example method 320 of performing image processing by an image processing service 112. At block 322, the image processing service 112 receives compressed, encrypted image data from an imaging session at a healthcare location.

The image processing service 112 unencrypts the image data at block 324. In various examples, a microservice of the image processing service 112 may unencrypt the image data. In addition to unencrypting the data, the image processing service 112 may, in various examples, decode the compressed image data before analysis of the image data. In some examples, the cloud based image processing service 112 may, at block 324, generate unique fingerprints or identifiers for each frame of image data upon receipt at the cloud based image processing service 112. Such fingerprints may be used by the cloud image processing service 112 to track the order of images within the image data, to track users and/or devices accessing the cloud image processing service 112, to correlate annotations with specific frames of the image data, and the like.

The image processing service 112 generates unique identifiers for compressed images of the compressed image data at block 326. In various examples, the unique identifiers may be fingerprints or other metadata associated with the compressed images and may identify an order of the compressed images within the compressed image data. For example, the unique identifiers may be generated based on a clock at the image processing service 112, an order in which the compressed images are received at the processing service 112, a time at which the compressed images were created at the healthcare location, and the like. In some examples, unique identifiers or fingerprints may be generated at a healthcare location either before or after compressing and encrypting the image data for transmission to the image processing service 112. In such examples, the image processing service 112 may utilize the unique identifiers to track information about frames of the image data, such as when the frames are received at the image processing service 112.

At block 328, the image processing service 112 performs image analysis on the compressed image data. The image analysis may be performed by a microservice of the image processing service 112 specifically configured for the type of image analysis being requested. For example, where the images are provided for polyp detection, the image stream may be transmitted to a microservice for polyp detection. In some examples, the image analysis may be performed using a detector trained to detect a specific type of structure (e.g., a polyp). The detector may be, for example, a neural network trained on compressed image data. For example, the neural network may be trained using lossy data, such that the neural network is able to provide meaningful image analysis on such lossy data. In contrast, a network trained on uncompressed image data may not identify important elements in lossy images. Further, training on lossy data may be completed more quickly than training on uncompressed data.

In various examples, the result of the image analysis may be some annotation to the original image to help identify the desired structure. For example, the image processing service 112 may add flags to the image noting a location of interest, a bounding box enclosing a region of interest, a risk result, an indication that the physician has reached a certain part of the anatomy, and the like. In various examples, the user may choose, via the local compute resources 104, a particular type of annotation to be returned by the image processing service 112. In various examples, the image processing service 112 may generate fingerprints or identifiers for such annotations. For example, fingerprints or identifiers may be used to correlate annotations to particular frames or images of a stream of image data. The fingerprints or identifiers may be generated by the image processing service 112 when annotations are generated and may be based on fingerprints or identifiers associated with the frames or images.

The image processing service 112 encrypts the image analysis results at block 330. For example, the imaging processing service 112 may encrypt the annotation produced by the image processing service 112 using TLS specifications, such as HTTPS or WSS protocols. At block 332, the image processing service 112 streams the image analysis results to the healthcare location. In various examples, the image processing service 112 may utilize the fingerprints or identifiers generated for the annotations and/or the images may be used by the image processing service 112 to stream the image analysis results to the healthcare location. For example, identifiers may be used to make sure that the annotations are in a correct order when streamed to the healthcare location.

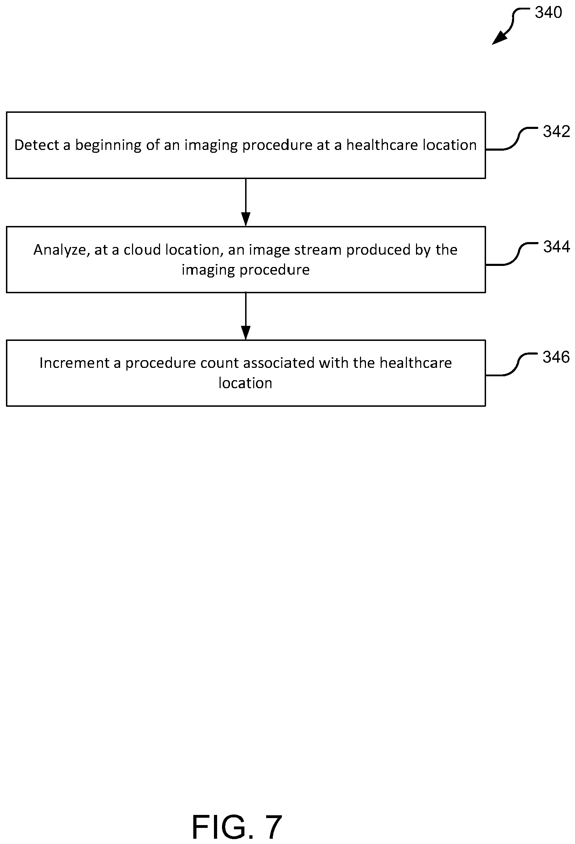

FIG. 7 is a flow diagram of an example method 340 of counting procedures originating from a healthcare facility utilizing an image processing service 112. Procedures may be counted by the image processing service 112 by tracking procedures associated with a user ID. The user ID may, in some examples, be provided with other credentials to the image processing service 112 at login.

The image processing service 112 detects a beginning of an imaging procedure at a healthcare location at block 342. In some examples, the image processing service 112 may detect a beginning of a procedure when an end user presses a button or actuates another mechanism to start a procedure. For example, a procedure may be started by pressing a button on the imaging device 102, pressing a foot pedal connected to the imaging device 102, and the like. In some examples, the image processing service 112 may automatically detect the beginning of a procedure using other metrics. For example, the image processing service 112 may detect the beginning of a procedure when the imaging device 102 is turned on a probe of the imaging device 102 is inside of the body.

The image processing service 112 analyzes, at a cloud location, an image stream produced by the imaging procedure at block 344. The image processing service 112 may begin analyzing and/or processing the image after the beginning of the procedure. Such image processing may be conducted using the method 320 of FIG. 6. In some examples, the image processing service 112 may, after detecting the beginning of a procedure, continually monitor for the an endpoint of the procedure. An endpoint may be detected based on several defined actions. For example, a user may mark the end of an imaging procedure by pressing an "end procedure" button at the imaging device 102. An endpoint may also be detected based on the images being received by the image processing service 112. For example, the image processing service 112 may detect that a procedure has ended when a probe of the imaging device 102 has been outside of the body for a certain amount of time (e.g., more than 2 minutes). In some examples, the image processing service 112 may also detect an endpoint after passage of a specific amount of time after the beginning of the procedure.

At block 346, the image processing service 112 increments a procedure count associated with the healthcare location. In some examples, the image processing service 112 may determine whether to increment a procedure count based on characteristics of the imaging procedure. For example, where a procedure length is less than two minutes, the image processing service 112 may not increment the counter. In some examples, the image processing service 112 may not increment the counter when a procedure is triggered but the imaging device 102 is not turned on or does not transmit data to the image processing service. Accordingly, the image processing service 112 may increment the procedure count for genuine imaging procedures.

In some examples, along with incrementing a procedure count, the image processing service 112 may track other information associated with the imaging procedure that may be used to track usage of the service by a healthcare location. For example, the image processing service 112 may track total procedure time. In some examples, the image processing service 112 may further display counter metrics (e.g., by transmitting metrics to the local compute resources 104 for display via the display 106) after incrementing the procedure count. For example, the image processing service 112 may transmit a number of procedures remaining on a prepaid plan, a number of procedures used over a time period, and the like.

In accordance with the above description, the image processing service 112 provides real time image analysis, enabling the use of high power compute resources during imaging procedures, without the associated costs and upkeep at healthcare locations. The image processing service 112 is further available for access by multiple healthcare locations and may be scaled in or out based on actual usage, providing efficient usage of computing resources. For example, the image processing service 112 may provide a low latency and low bandwidth connection between local compute resources 104 and the cloud based image processing service 112. Such a low latency and low bandwidth connection allows for image analysis taking advantage of advanced computing resources available in cloud environments while providing real time image analysis at a healthcare location. The image processing service 112 may further be structured including various microservices, providing for message ordering (e.g., tasks received first may be completed first), task distribution among microservices to balance workloads, and automatic scaling of the image processing service 112. The image processing service 112 may provide other advantages, such as security and encryption, asynchronous data streaming, data anonymization, monitoring of network performance, and the like.

The description of certain embodiments included herein is merely exemplary in nature and is in no way intended to limit the scope of the disclosure or its applications or uses. In the included detailed description of embodiments of the present systems and methods, reference is made to the accompanying drawings which form a part hereof, and which are shown by way of illustration specific to embodiments in which the described systems and methods may be practiced. These embodiments are described in sufficient detail to enable those skilled in the art to practice presently disclosed systems and methods, and it is to be understood that other embodiments may be utilized, and that structural and logical changes may be made without departing from the spirit and scope of the disclosure. Moreover, for the purpose of clarity, detailed descriptions of certain features will not be discussed when they would be apparent to those with skill in the art so as not to obscure the description of embodiments of the disclosure. The included detailed description is therefore not to be taken in a limiting sense, and the scope of the disclosure is defined only by the appended claims.

From the foregoing it will be appreciated that, although specific embodiments of the invention have been described herein for purposes of illustration, various modifications may be made without deviating from the scope of the invention.

The particulars shown herein are by way of example and for purposes of illustrative discussion of the preferred embodiments of the present invention only and are presented in the cause of providing what is believed to be the most useful and readily understood description of the principles and conceptual aspects of various embodiments of the invention. In this regard, no attempt is made to show structural details of the invention in more detail than is necessary for the fundamental understanding of the invention, the description taken with the drawings and/or examples making apparent to those skilled in the art how the several forms of the invention may be embodied in practice.

As used herein and unless otherwise indicated, the terms "a" and "an" are taken to mean "one", "at least one" or "one or more". Unless otherwise required by context, singular terms used herein shall include pluralities and plural terms shall include the singular.

Unless the context clearly requires otherwise, throughout the description and the claims, the words 'comprise', 'comprising', and the like are to be construed in an inclusive sense as opposed to an exclusive or exhaustive sense; that is to say, in the sense of "including, but not limited to". Words using the singular or plural number also include the plural and singular number, respectively. Additionally, the words "herein," "above," and "below" and words of similar import, when used in this application, shall refer to this application as a whole and not to any particular portions of the application.

Of course, it is to be appreciated that any one of the examples, embodiments or processes described herein may be combined with one or more other examples, embodiments and/or processes or be separated and/or performed amongst separate devices or device portions in accordance with the present systems, devices and methods.

Finally, the above discussion is intended to be merely illustrative of the present system and should not be construed as limiting the appended claims to any particular embodiment or group of embodiments. Thus, while the present system has been described in particular detail with reference to exemplary embodiments, it should also be appreciated that numerous modifications and alternative embodiments may be devised by those having ordinary skill in the art without departing from the broader and intended scope of the present system as set forth in the claims that follow. Accordingly, the specification and drawings are to be regarded in an illustrative manner and are not intended to limit the scope of the appended claims.

The present disclosure also includes the following numbered clauses, which set out feature combinations that are useful for understanding the present disclosure:
1. A method comprising:
   receiving encrypted compressed image data from an imaging session at a healthcare location;
      unencrypting the compressed image data;
   generating unique identifiers for compressed images of the compressed image data, wherein the unique identifiers identify an order of the compressed images within the compressed image data;
   identifying a characteristic of interest in the compressed image data;
   annotating the compressed images of the compressed image data reflecting the identified characteristic of interest to generate computer annotations; and
   streaming the computer annotations to the healthcare location as the imaging session is in progress using the unique identifiers.
2. The method of clause 1, wherein the compressed image data is lossy image data.
3. The method of clause 1, further comprising:
   displaying, at the healthcare location, the computer annotations and image data collected during the imaging session at the healthcare location.
4. The method of clause 1, wherein the characteristic of interest is identified using a machine learning model trained using compressed image data.
5. The method of clause 1, wherein annotating the compressed image data comprises creating the computer annotations, the computer annotations specifying where in the image data the identified characteristic of interest is located.
6. The method of clause 1, wherein the compressed, encrypted image data is compressed and encrypted by local compute resources collocated with an imaging device used to collect the image data during the imaging session.
7. The method of clause 6, wherein the compressed, encrypted image data is compressed using an autoencoder network located at least partially at the local compute resources.
8. A system comprising:
   local computing resources configured to compress image data received from an imaging device, the image data collected during an imaging procedure; and
   an image processing service hosted in a cloud computing environment configured to:
      receive compressed image data from the local computing resources,
      generate unique identifiers for compressed images of the compressed image data, wherein the unique identifiers identify an order of the compressed images within the compressed image data.
      analyze the compressed image data to provide computer annotations to the compressed images of the compressed image data, and
      transmit the computer annotations to the local computing resources for display as the imaging procedure is ongoing using the unique identifiers.
9. The system of clause 8, wherein the local computing resources are further configured to split the image data received from the imaging device into a first stream and a second stream, wherein the compressed image data is created from the first stream of the image data.
10. The system of clause 9, wherein the local computing resources are further configured to display the second stream of image data and the computer annotations together as the imaging procedure is ongoing.
11. The system of clause 10, wherein the local computing resources are further configured to display the computer annotations responsive to a determination that the computer annotations were received at the local computing resources before a threshold time after sending the compressed image data to the image processing service.
12. The system of clause 8, wherein the image processing service includes a machine learning model trained using compressed image data, wherein the compressed images are analyzed using the machine learning model.
13. The system of clause 8, wherein the image processing service is further configured to generate second unique identifiers for the computer annotations based at least on the unique identifiers of the compressed images.
14. The system of clause 8, wherein the local computing resources are further configured to anonymize the image data, wherein anonymizing the image data comprises removing one or more pieces of identifying information from the image data.
15. A method comprising:
   detecting a beginning of an imaging procedure at a healthcare location;
   generating, at a cloud location, unique identifiers for images in an image stream produced during the procedure;
   analyzing, at a cloud location, the images using the unique identifiers to determine an order of the images within the image stream; and
   incrementing a procedure count associated with the healthcare location.
16. The method of clause 15, wherein detecting the beginning of the imaging procedure comprises detecting selection of a procedure beginning marker at an imaging device used in the imaging procedure.
17. The method of clause 15, further comprising:
   identifying the healthcare location from a plurality of healthcare locations based on an identifier of local compute resources collocated with an imaging device used in the imaging procedure, wherein the identifier is associated with the healthcare location.
18. The method of clause 15, further comprising:
   determining, prior to analyzing the image stream, that the image stream is produced from a valid imaging procedure.
19. The method of clause 18, wherein determining that the image stream is produced from a valid imaging procedure comprises detecting that images of the image stream are collected while an imaging device used in the imaging procedure is inside of a patient's body.
20. The method of clause 15, wherein analyzing the image stream produced during the imaging procedure comprises analyzing a compressed image stream.

## Claims

1. A method comprising:
receiving encrypted compressed image data from an imaging session at a healthcare location;
unencrypting the compressed image data;
generating unique identifiers for compressed images of the compressed image data, wherein the unique identifiers identify an order of the compressed images within the compressed image data;
identifying a characteristic of interest in the compressed image data;
annotating the compressed images of the compressed image data reflecting the identified characteristic of interest to generate computer annotations; and
streaming the computer annotations to the healthcare location as the imaging session is in progress using the unique identifiers.

2. The method of claim 1, wherein the compressed image data is lossy image data.

3. The method of claim 1 or claim 2, further comprising:
displaying, at the healthcare location, the computer annotations and image data collected during the imaging session at the healthcare location.

4. The method of any of claims 1 to 3, wherein:
the characteristic of interest is identified using a machine learning model trained using compressed image data; and/or
annotating the compressed image data comprises creating the computer annotations, the computer annotations specifying where in the image data the identified characteristic of interest is located.

5. The method of any of claims 1 to 4, wherein the compressed, encrypted image data is compressed and encrypted by local compute resources collocated with an imaging device used to collect the image data during the imaging session;
wherein the compressed, encrypted image data is optionally compressed using an autoencoder network located at least partially at the local compute resources.

6. A system comprising:
local computing resources configured to compress image data received from an imaging device, the image data collected during an imaging procedure; and
an image processing service hosted in a cloud computing environment configured to:
receive compressed image data from the local computing resources,
generate unique identifiers for compressed images of the compressed image data, wherein the unique identifiers identify an order of the compressed images within the compressed image data.
analyze the compressed image data to provide computer annotations to the compressed images of the compressed image data, and
transmit the computer annotations to the local computing resources for display as the imaging procedure is ongoing using the unique identifiers.

7. The system of claim 6, wherein the local computing resources are further configured to split the image data received from the imaging device into a first stream and a second stream, wherein the compressed image data is created from the first stream of the image data;
wherein the local computing resources are optionally further configured to display the second stream of image data and the computer annotations together as the imaging procedure is ongoing, and wherein the local computing resources are optionally further configured to display the computer annotations responsive to a determination that the computer annotations were received at the local computing resources before a threshold time after sending the compressed image data to the image processing service.

8. The system of claim 6 or claim 7, wherein the image processing service includes a machine learning model trained using compressed image data, wherein the compressed images are analyzed using the machine learning model.

9. The system of any of claims 6 to 8, wherein the unique identifiers are first unique identifiers and the image processing service is further configured to generate second unique identifiers for the computer annotations based at least on the unique identifiers of the compressed images, wherein the first unique identifiers and the second unique identifiers are generated at the healthcare location or at a cloud location.

10. The system of any of claims 6 to 9, wherein the local computing resources are further configured to anonymize the image data, wherein anonymizing the image data comprises removing one or more pieces of identifying information from the image data.

11. A method comprising:
detecting a beginning of an imaging procedure at a healthcare location;
generating unique identifiers for images in an image stream produced during the procedure;
analyzing, at a cloud location, the images using the unique identifiers to determine an order of the images within the image stream; and
incrementing a procedure count associated with the healthcare location.

12. The method of claim 11, wherein detecting the beginning of the imaging procedure comprises detecting selection of a procedure beginning marker at an imaging device used in the imaging procedure.

13. The method of claim 11 or claim 12, further comprising:
identifying the healthcare location from a plurality of healthcare locations based on an identifier of local compute resources collocated with an imaging device used in the imaging procedure, wherein the identifier is associated with the healthcare location.

14. The method of any of claims 11 to 13, further comprising:
determining, prior to analyzing the image stream, that the image stream is produced from a valid imaging procedure;
wherein determining that the image stream is produced from a valid imaging procedure optionally comprises detecting that images of the image stream are collected while an imaging device used in the imaging procedure is inside of a patient's body.

15. The method of any of claims 11 to 14, wherein:
analyzing the image stream produced during the imaging procedure comprises analyzing a compressed image stream; and/or
the unique identifiers are generated at one of the healthcare location or at a cloud location.
